# EUROPÄISCHE PATENTSCHRIFT

(11) **EP 2 892 866 B1**
(45) Veröffentlichungstag und Bekanntmachung des Hinweises auf die Patenterteilung: **20.07.2016**
(21) Anmeldenummer: 13758882.8
(22) Anmeldetag: 06.09.2013
(51) Int. Cl.: C07C 29/36, C07C 33/20

(54) **VERFAHREN ZUR HERSTELLUNG VON 2-METHYL-4-PHENYL-BUTAN-2-OL**
METHOD FOR PREPARING 2-METHYL-4-PHENYLBUTAN-2-OL
PROCÉDÉ DE FABRICATION DE 2-MÉTHYL-4-PHÉNYL-BUTANE-2-OL

(30) Priorität: 07.09.2012 EP 12183476
(43) Veröffentlichungstag der Anmeldung: 15.07.2015
(73) Patentinhaber: BASF SE, 67056 Ludwigshafen am Rhein (DE)
(72) Erfinder: RÜDENAUER, Stefan, 67549 Worms (DE)
(74) Vertreter: BASF IP Association
(86) Internationale Anmeldenummer: PCT/EP2013/068432
(87) Internationale Veröffentlichungsnummer: WO 2014/037483

(56) Entgegenhaltungen:
- WO-A2-2011/117360
- JP-A- 2000 103 754
- F. H. NORTON ET AL: JOURNAL OF THE AMERICAN CHEMICAL SOCIETY, Bd. 58, Nr. 11, 1. November 1936 (1936-11-01), Seiten 2147-2150, XP055053547, ISSN: 0002-7863, DOI: 10.1021/ja01302a017
- ERDELYI B ET AL: "Stereoselective production of (S)-1-aralkyl- and 1-arylethanols by freshly harvested and lyophilized yeast cells", TETRAHEDRON ASYMMETRY, PERGAMON PRESS LTD, OXFORD, GB, Bd. 17, Nr. 2, 23. Januar 2006 (2006-01-23), Seiten 268-274, XP024962551, ISSN: 0957-4166, DOI: 10.1016/J.TETASY.2005.12.025 [gefunden am 2006-01-23]

## Beschreibung

Die vorliegende Erfindung betrifft ein Verfahren zur Herstellung von 2-Methyl-4-phenyl-butan-2-ol.

2-Methyl-4-phenyl-butan-2-ol, das auch als Dimethylphenylethylcarbinol oder "Carbinol Muguet" bezeichnet wird, ist ein Riechstoff mit einem blumigen Geruch, der etwas grün und krautig ist und in Richtung Hyacinthen- und Lilienduft geht (WO 2004/076393 A1). 2-Methyl-4-phenyl-butan-2-ol wird zur Verbesserung des Geruchs und/oder Geschmacks eines Produkts oder auch zur Maskierung des Eigengeruchs und/oder - geschmacks eines Produkts verwendet. Ferner ist 2-Methyl-4-phenyl-butan-2-ol eine Vorstufe bei der Herstellung weitere Duftstoffe wie 4-Cyclohexyl-2-methyl-2-butanol, das auch als Coranol bezeichnet wird und einen Maiglöckchenduft besitzt.

Ein Verfahren zur Herstellung von 4-Cyclohexyl-2-methyl-2-butanol, bei dem als Zwischenprodukt 2-Methyl-4-phenyl-butan-2-ol entsteht, wurde von Ebel et al. (WO 2011/117360) beschrieben. Dabei wird 2-Methyl-4-phenyl-butan-2-ol durch die Umsetzung von Styrol mit Isopropanol gebildet. Ein Verfahren zur Herstellung von Dimethylphenylethylcarbinol mittels Umsetzung von Methylmagnesiumchlorid mit Benzylaceton wurde von Yoichi et al. (JP 2000103754 A) beschrieben.

Gegenstand der vorliegenden Erfindung ist ein alternatives Verfahren zur Herstellung von 2-Methyl-4-phenyl-butan-2-ol aus leicht zugänglichen Ausgangsstoffen. Bei diesem Verfahren wird ein Benzylmagnesiumhalogenid mit Isobutylenoxid umgesetzt. Das Benzylmagnesiumhalogenid ist vorzugsweise Benzylmagnesiumbromid oder Benzylmagnesiumchlorid, insbesondere Benzylmagnesiumbromid.

Die Herstellung von 2-Methyl-4-phenyl-butan-2-ol aus einem Benzylmagnesiumhalogenid (I) und Isobutylenoxid lässt sich durch das folgende Schema darstellen, wobei X ein Halogen, insbesondere Chlor, Brom oder Iod, und bevorzugt Brom ist:

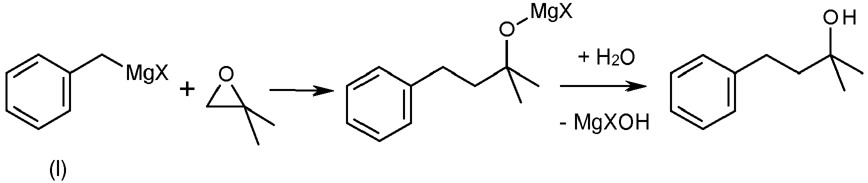

Das Benzylmagnesiumhalogenid (I) kann auf bekannte Weise aus Magnesium und einem Benzylhalogenid (II) hergestellt werden.

Die Herstellung des Benzylmagnesiumhalogenids (I) lässt sich durch das folgende Schema darstellen, wobei X ein Halogen, insbesondere Clor, Brom oder Iod, und bevorzugt Brom ist:

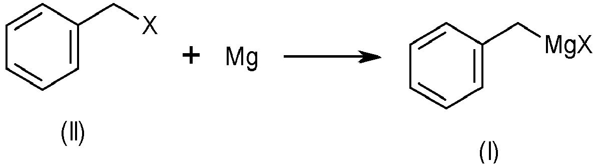

Es ist vorteilhaft, Magnesium im stöchiometrischen Überschuss, bezogen auf das Benzylhalogenid (II), einzusetzen. Bevorzugt werden 1 bis 10 mol, vorzugsweise 1,5 bis 5 mol, stärker bevorzugt 2 bis 3 mol und insbesondere etwa 2,5 mol Magnesium pro mol Benzylhalogenid verwendet.

Zum Anätzen des Magnesiums kann Iod zugegeben werden.

Die Reaktion verläuft exotherm. Die Temperatur des Reaktionsansatzes wird bevorzugt zwischen 0°C und 70°C, insbesondere zwischen 40°C und 60°C gehalten. Dies kann u.a. durch entsprechendes Einstellen der Zugabegeschwindigkeit des Benzylhalogenids (II) zum vorgelegten Magnesium erreicht werden.

Die Herstellung des Benzylmagnesiumhalogenids (I) findet geeigneterweise in einem Verdünnungsmittel in Abwesenheit von Wasser statt. Als Verdünnungsmittel können die nachfolgend genannten inerten Lösungsmittel verwendet werden.

Die Umsetzung des Benzylmagnesiumhalogenids (I) mit Isobutylenoxid kann bei verschiedenen stöchiometrischen Verhältnissen dieser Ausgangsstoffe erfolgen. Benzylmagnesiumhalogenid (I) kann, bezogen auf das Isobutylenoxid, sowohl im stöchiometrischen Überschuss als auch im stöchiometrischen Unterschuss oder auch in stöchiometrisch gleicher Menge vorliegen. Bevorzugt werden 0,5 bis 5 mol, vorzugsweise 1,5 bis 2,5 mol und insbesondere 1 bis 2 mol Benzylmagnesiumhalogenid pro mol Isobutylenoxid verwendet.

Bevorzugt wird das Benzylmagnesiumhalogenid (I) vorgelegt und Isobutylenoxid zugegeben. Es kann aber auch Isobutylenoxid vorgelegt und das Benzylmagnesiumhalogenid (I) zugegeben werden.

Die Reaktion kann sowohl in Batch-Fahrweise als auch in Semi-Batch-Fahrweise oder auch in kontinuierlicher Form erfolgen.

Geeigneterweise findet die Reaktion von Benzylmagnesiumhalogenid (I) mit Isobutylenoxid in einem Verdünnungsmittel in Abwesenheit von Wasser statt. Als Verdünnungsmittel sind unter den Reaktionsbedingungen inerte Lösungsmittel geeignet, insbesondere Ether, wie z.B. Tetrahydrofuran, 2-Methyltetrahydrofuran, 1,4-Dioxan, Diethylether, Diisopropylether und/oder Dibutylether; aliphatische Kohlenwasserstoffe, wie z.B. Ligroin, Heptan und/oder Oktan; und aromatischen Kohlenwasserstoffe, wie z.B. Benzol, Toluol und/oder Xylol, und Gemische davon. Bevorzugt wird ein Ether bzw. Ethergemisch, besonders bevorzugt Tetrahydrofuran und/oder 2-Methylhydrofuran, verwendet. Das Verdünnungsmittel ist vorzugweise im Wesentlichen wasserfrei.

Die Umsetzung erfolgt vorzugsweise in Gegenwart eines Epoxid-Ringöffnungskatalysators. Cu(I)-Verbindungen sind bevorzugte Epoxid-Ringöffnungskatalysatoren. Geeignete Cu(I)-Verbindungen sind CuCI, CuBr, Cul und/oder CuCN, wovon Cul bevorzugt ist. Die Cu(I)-Verbindung wird bevorzugt in Mengen von 0,05 bis 0,3 mol, von 0,07 bis 0,15 mol und insbesondere etwa 0,1 mol pro mol Isobutylenoxid verwendet. Vorzugsweise gibt man die Cu(I)-Verbindung zum vorgelegten Benzylmagnesiumhalogenid. Anschließend kann man das Isobutylenoxid zugeben.

Die Umsetzung von Benzylmagnesiumhalogenid (I) mit Isobutylenoxid findet bevorzugt bei -20°C bis +10°C, insbesondere bei -10°C bis 0°C, statt. Diese Temperatur kann durch Kühlung der Ausgangsstoffe bzw. des Reaktionsgefäßes und auch durch entsprechendes Einstellen der Geschwindigkeit erreicht werden, mit der die Ausgangsstoffe vereinigt werden. So kann dazu etwa die Zugabegeschwindigkeit des Isobutylenoxids zum vorgelegten Benzylmagnesiumhalogenid eingestellt werden, z.B. kann eine kontinuierliche Zugabe des Isobutylenoxids über einen Zeitraum von 0,5 bis 1,5 h erfolgen.

Nach Beendigung der Reaktion erfolgt die Aufarbeitung der Reaktionslösung durch Hydrolyse, beispielsweise mit Wasser oder auch mit wässriger Säure oder wässriger Base. So können zur Aufarbeitung mit wässriger Säure anorganische Säuren, wie z.B. Salzsäure oder Ammoniumchlorid, oder auch organische Säuren verwendet werden. Zur Aufarbeitung mit wässriger Base sind beispielsweise wässrige Lösungen von Natriumhydrogencarbonat, Natriumcarbonat, Kaliumhydrogencarbonat, Kaliumcarbonat oder Natriumhydroxid verwendbar. Das Zielprodukt (gebildetes 2-Methyl-4-phenyl-butan-2-ol) kann durch Extraktion von der wässrigen Phase getrennt und nach Trocknung der organischen Phase aus dieser durch Verfahren wie Destillation oder Schmelzkristallisation isoliert werden.

Ein Vorteil des erfindungsgemäßen Verfahrens besteht darin, dass die Herstellung des Benzylmagnesiumhalogenids (I) und die Umsetzung desselben mit Isobutylenoxid ohne zwischengeschaltete Schritte in einem Reaktionsansatz und im selben Verdünnungsmittel erfolgen können. Dementsprechend betrifft die vorliegende Erfindung auch ein Verfahren, bei dem in einem ersten Schritt ein Benzylhalogenid (II), wie beschrieben, mit Magnesium umgesetzt wird und das entstandene Benzylmagnesiumhalogenid anschließend, wie beschrieben, in Gegenwart einer Cu(I)-Verbindung mit Isobutylenoxid umgesetzt wird.

Das mit dem erfindungsgemäßen Verfahren erhaltene 2-Methyl-4-phenyl-butan-2-ol kann als Duft- oder Aromastoff eingesetzt werden, insbesondere in kosmetischen Mitteln, Textilwaschmitteln und Reinigungsmitteln für harte Flächen.

Beispiele für derartige kosmetische Mittel umfassen grundsätzlich alle kosmetischen Zusammensetzungen, die üblicherweise Duftstoffe enthalten. Hierzu zählen z.B. Eaux-de-Parfum, Eaux-de-Toilette, Eaux-de-Cologne, After-Shave Produkte wie Lotionen und Cremes, Pre-Shave Produkte, parfümierte Erfrischungstücher, Enthaarungs-Cremes and Lotionen, Bräunungscremes und -lotionen, Haarpflegemittel wie Shampoos, Haarspülungen, Haarfestiger, Haargele, Haartönungsmittel, Haarwachse, Haarsprays, Schaumfestiger, Haarmousses, Spitzenfluids, Egalisierungsmittel für Dauerwellen, Haarfärbe- und -bleichmittels oder "Hot-Oil-Treatments", weiterhin Hautreinigungsmittel wie Seifen, Waschgele, Duschgele, Körperpflegemittel wie Cremes, Öle, Lotionen und dergleichen für Haut, insbesondere Produkte zur Pflege der Hände, des Gesichts oder der Füße, Sonnenschutzmittel, Deodorantien and Antiperspirantien, Hautdesinfektionsmittel, Insekten-Repellents sowie dekorative kosmetische Produkte. Je nach Anwendungsgebiet können die kosmetischen Zusammensetzungen als wässrige oder alkoholische Flüssigkeit, Öl, (Aerosol-)Spray, (Aerosol-)Schaum, Mousse, Gel, Gelspray, Creme, Lotion, Puder, Tabs, oder Wachs formuliert werden.

Zu Wasch- bzw. Reinigungsmitteln, in denen der mit dem erfindungsgemäßen Verfahren erhaltene 2-Methyl-4-phenyl-butan-2-ol enthalten sein kann, zählen Mittel zur Reinigung und/oder Desinfektion von Oberflächen, wie beispielsweise Haushaltsreiniger, Neutralreiniger, Toilettenreiniger, Bodenreiniger, Teppichreiniger, Fensterreiniger, Polituren, Möbelpflegeprodukte, flüssige und feste Geschirrspülmittel, flüssige und feste Maschinenspülmittel, weiterhin Mittel zum Reinigen oder Behandeln von Textilien wie feste, halbfeste oder flüssige Textilwaschmittel, Wäschenachbehandlungsmittel, Weichspüler, Bügelhilfsmittel, Textilerfrischer, Gewebe-Prekonditioniermittel, Waschseifen, Waschtabletten und dergleichen.

Ferner kann der mit dem erfindungsgemäßen Verfahren erhaltene 2-Methyl-4-phenyl-butan-2-ol als Duftstoffbestandteil in anderen duftstoffhaltigen Produkten wie Luftreinigern, Lampenölen, Kerzen, Raumluftverbesserern, WC-Steinen und dergleichen eingesetzt werden.

Die Erfindung wird anhand der folgenden Beispiele näher erläutert:

### Beispiel 1: Herstellung von Benzylmagnesiumbromid

Ein 6I-Reaktor (HWS Labortechnik Mainz) mit Außenmantelbeheizung wurde mit Argon inertisiert. Es wurde eine Suspension von 88,71 g Magnesium in 1500 ml Tetrahydrofuran im Kolben vorgelegt und unter Rühren 0,50 g Iod zugegeben. Dabei färbte sich die Suspension bräunlich-gelblich. Anschließend wurden über einen Zeitraum von 165 min nach und nach 250,00 g Benzylbromid über einen Tropftrichter zugegeben. Die Suspension entfärbte sich dabei. Nach anfänglichem Erwärmen des Kolbeninhalts auf 56°C verlief die Reaktion exotherm. Die Geschwindigkeit der Benzylbromidzugabe wurde so eingestellt, dass die Temperatur des Kolbeninhalts zwischen 46°C und 56°C verblieb. Nach erfolgter Zugabe des Benzylbromids und Abkühlung des Kolbeninhalts auf Raumtemperatur wurde der Überstand in einen zweiten inertisierten Kolben dekantiert.

### Beispiel 2: Herstellung von 2-Methyl-4-phenyl-butan-2-ol

Zum gemäß Beispiel 1 erhaltenen Überstand wurden 13,92 g Kupfer(I)-iodid hinzugegeben und der Kolben auf -10°C abgekühlt. Über einen Zeitraum von einer Stunde wurden anschließend nach und nach 52,70 g Isobutylenoxid über einen Tropftrichter zugegeben und die Temperatur zwischen -10°C und -6°C gehalten. Dabei wurde eine leichte Exothermie beobachtet. Es wurde zwei Stunden bei 0°C nachgerührt und dann eine Probe für die Analyse per Gaschromatograph genommen. Diese wurde mit Ammoniumchloridlösung aufgearbeitet. Das Reaktionsgemisch wurde mit 400 ml gesättigter Ammoniumchlorid-Lösung aufgearbeitet und 200 ml Toluol zugesetzt. Die organische Phase wurde abgetrennt und zweimal mit je 400 ml gesättigter Ammoniumchlori-Lösung gewaschen. Nach Trocknung über Magnesiumsulfat wurde das Produkt durch Destillation im Vakuum mit einer Reinheit von > 97 % erhalten. In Summe wurden 88,9 g 2-Methyl-4-phenyl-butan-2-ol erhalten, was einer Ausbeute von 74 % entspricht.

## Patentansprüche

1. Verfahren zur Herstellung von 2-Methyl-4-phenyl-butan-2-ol, bei dem ein Benzylmagnesiumhalogenid mit Isobutylenoxid umgesetzt wird.

2. Verfahren nach Anspruch 1, wobei das Benzylmagnesiumhalogenid Benzylmagnesiumbromid oder Benzylmagnesiumchlorid ist.

3. Verfahren nach Anspruch 1 oder 2, wobei 0,5 bis 5 mol Benzylmagnesiumhalogenid pro mol Isobutylenoxid verwendet werden.

4. Verfahren nach einem der vorhergehenden Ansprüche, wobei die Umsetzung in Gegenwart mindestens einer Cu(I)-Verbindung erfolgt.

5. Verfahren nach Anspruch 4, wobei die Cu(I)-Verbindung Kupfer(I)-iodid ist.

6. Verfahren nach Anspruch 4 oder 5, wobei 0,05 bis 0,3 mol Cu(I)-Verbindung pro mol Isobutylenoxid verwendet werden.

7. Verfahren nach einem der vorhergehenden Ansprüche, wobei das Benzylmagnesiumhalogenid durch Umsetzung eines Benzylhalogenids mit Magnesium erhalten ist.

8. Verfahren nach Anspruch 7, wobei 1 bis 10 mol Magnesium pro mol Benzylhalogenid verwendet werden.

9. Verfahren nach Anspruch 7 oder 8, wobei die Umsetzung des Benzylhalogenids mit Magnesium und die Umsetzung des entstandenen Benzylmagnesiumhalogenids mit Isobutylenoxid im selben Verdünnungsmittel stattfinden.

10. Verfahren nach Anspruch 9, wobei das Verdünnungsmittel ein Ether ist.

11. Verfahren nach Anspruch 10, wobei der Ether Tetrahydrofuran oder 2-Methyltetrahydrofuran ist.

## Claims

1. A method for preparing 2-methyl-4-phenylbutan-2-ol by reaction of a benzylmagnesium halide with isobutylene oxide.

2. The method according to claim 1, in which the benzylmagnesium halide is benzylmagnesium bromide or benzylmagnesium chloride.

3. The method according to claim 1 or 2, in which 0.5 to 5 mol of benzylmagnesium halide is used per mole of isobutylene oxide.

4. The method according to any of the preceding claims, in which the reaction is carried out in the presence of at least one Cu(I) compound.

5. The method according to claim 4, in which the Cu(I) compound is copper(I) iodide.

6. The method according to claim 4 or 5, in which 0.05 to 0.3 mol of Cu(I) compound is used per mole of isobutylene oxide.

7. The method according to any of the preceding claims, in which the benzylmagnesium halide is obtained by reaction of a benzyl halide with magnesium.

8. The method according to claim 7, in which 1 to 10 mol of magnesium is used per mole of benzyl halide.

9. The method according to claim 7 or 8, in which the reaction of the benzyl halide with magnesium and the reaction of the benzylmagnesium halide thus produced with isobutylene oxide take place in the same diluent.

10. The method according to claim 9, in which the diluent is an ether.

11. The method according to claim 10, in which the ether is tetrahydrofuran or 2-methyltetrahydrofuran.

## Revendications

1. Procédé de préparation de 2-méthyl-4-phényl-butan-2-ol, dans lequel on fait réagir un halogénure de benzylmagnésium avec de l'oxyde d'isobutylène.

2. Procédé selon la revendication 1, dans lequel l'halogénure de benzylmagnésium est du bromure de benzylmagnésium ou du chlorure de benzylmagnésium.

3. Procédé selon la revendication 1 ou 2, dans lequel on utilise 0,5 à 5 moles d'halogénure de benzylmagnésium par mole d'oxyde d'isobutylène.

4. Procédé selon l'une quelconque des revendications précédentes, dans lequel la réaction s'effectue en présence d'au moins un composé de Cu(I).

5. Procédé selon la revendication 4, dans lequel le composé de Cu(I) est de l'iodure de cuivre (I).

6. Procédé selon la revendication 4 ou 5, dans lequel on utilise de 0,05 à 0,3 mole de composé de Cu (I) par mole d'oxyde d'isobutylène.

7. Procédé selon l'une quelconque des revendications précédentes, dans lequel l'halogénure de benzylmagnésium est obtenu par réaction d'un halogénure de benzyle avec du magnésium.

8. Procédé selon la revendication 7, dans lequel on utilise 1 à 10 moles de magnésium par mole d'halogénure de benzyle.

9. Procédé selon la revendication 7 ou 8, dans lequel la réaction de l'halogénure de benzyle avec du magnésium et la réaction de l'halogénure de benzylmagnésium obtenu avec de l'oxyde d'isobutylène ont lieu dans le même diluant.

10. Procédé selon la revendication 9, dans lequel le diluant est de l'éther.

11. Procédé selon la revendication 10, dans lequel l'éther est du tétrahydrofurane ou du 2-méthyl-tétrahydrofurane.
